# EUROPEAN PATENT APPLICATION

(11) **EP 0 947 546 A1**
(43) Date of publication of application: **06.10.1999**
(21) Application number: 99202148.5
(22) Date of filing: 02.07.1999
(51) Int. Cl.: C08K 5/3492, C08K 5/3435, C08K 5/00, C07D 401/14

(54) **Blends of sterically hindered amines**

(71) Applicant: Ciba Specialty Chemicals Holding Inc., 4057 Basel (CH)
(72) Inventor: Gugumus, François, 4123 Allschwil (CH)

(57) **Abstract**

A mixture comprising two different sterically hindered amines one of which is a compound of the formula 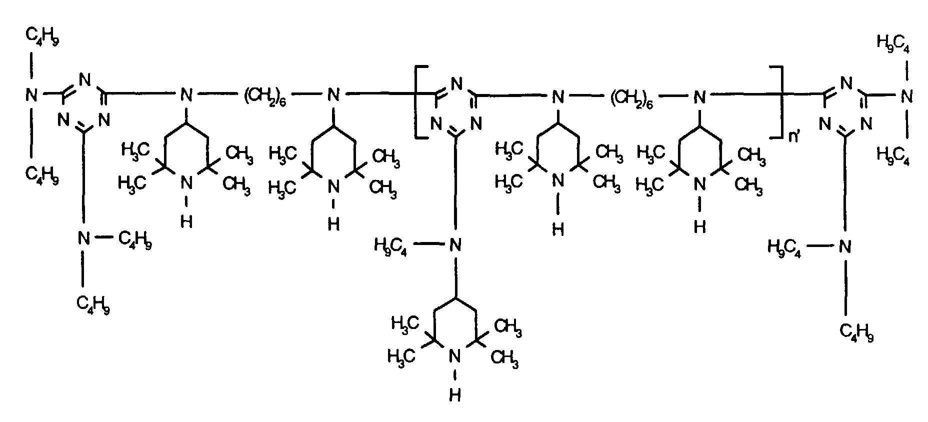 wherein n 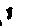 is a number from 2 to 14.

## Description

Mixtures containing a component α) and a component β) as defined below are particularly useful for stabilizing an organic material against degradation induced by light, heat or oxidation. The organic material is preferably a synthetic polymer, in particular a polyolefin. Examples of organic materials which can be stabilized are disclosed in EP-A-782,994 on page 19, line 1 to page 21, line 3.

**Component** α**)** is for example one of the mixtures disclosed in claim 10 or 38 or one of the products described in Examples 1 to 12 or Examples D-1 to D-5 of EP-A-782,994.

**Component** β**)** may be one of the sterically hindered amine compounds disclosed in EP-A-916,698, page 2, line 35 to page 40, line 55,

The product of Example 10 of EP-A-782,994 (**Component** α**)**) which corresponds to the formula 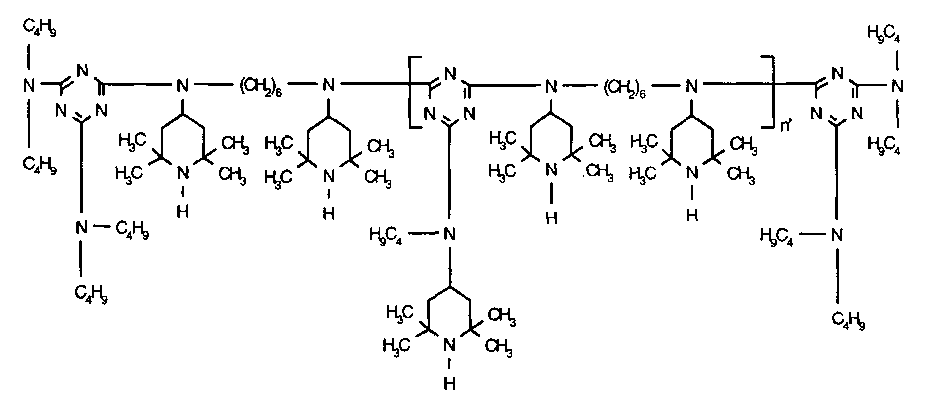 wherein n 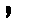 indicates that there are repetitive units in the molecule (n 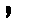 is e.g. a number from 2 to 14), is preferably used in combination with one of the following compounds (**Component** β**)**):
**1)** 4-hydroxy-2,2,6,6-tetramethylpiperidine
**2)** 1-allyl-4-hydroxy-2,2,6,6-tetramethylpiperidine
**3)** 1-benzyl-4-hydroxy-2,2,6,6-tetramethylpiperidine
**4)** 1-(4-tert-butyl-2-butenyl)-4-hydroxy-2,2,6,6-tetramethylpiperidine
**5)** 4-stearoyloxy-2,2,6,6-tetramethylpiperidine
**6)** 1-ethyl-4-salicyloyloxy-2,2,6,6-tetramethylpiperidine
**7)** 4-methacryloyloxy-1,2,2,6,6-pentamethylpiperidine
**8)** 1,2,2,6,6-pentamethylpiperidin-4-yl β-(3,5-di-tert-butyl-4-hydroxyphenyl)propionate
**9)** di(1-benzyl-2,2,6,6-tetramethylpiperidin-4-yl) maleate
**10)** di(2,2,6,6-tetramethylpiperidin-4-yl) succinate
**11)** di(2,2,6,6-tetramethylpiperidin-4-yl) glutarate
**12)** di(2,2,6,6-tetramethylpiperidin-4-yl) adipate
**13)** di(2,2,6,6-tetramethylpiperidin-4-yl) sebacate
**14)** di(1,2,2,6,6-pentamethylpiperidin-4-yl) sebacate
**15)** di(1,2,3,6-tetramethyl-2,6-diethyl-piperidin-4-yl) sebacate
**16)** di(1-allyl-2,2,6,6-tetramethylpiperidin-4-yl) phthalate
**17)** 1-hydroxy-4-β-cyanoethoxy-2,2,6,6-tetramethylpiperidine
**18)** 1-acetyl-2,2,6,6-tetramethylpiperidin-4-yl acetate
**19)** tri(2,2,6,6-tetramethylpiperidin-4-yl) trimellitate
**20)** 1-acryloyl-4-benzyloxy-2,2,6,6-tetramethylpiperidine
**21)** di(2,2,6,6-tetramethylpiperidin-4-yl) diethylmalonate
**22)** di(1,2,2,6,6-pentamethylpiperidin-4-yl) dibutylmalonate
**23)** di(1,2,2,6,6-pentamethylpiperidin-4-yl) butyl(3,5-di-tert-butyl-4-hydroxybenzyl) malonate
**24)** di(1-octyloxy-2,2,6,6-tetramethylpiperidin-4-yl) sebacate
**25)** di(1-cyclohexyloxy-2,2,6,6-tetramethylpiperidin-4-yl) sebacate
**26)** hexane-1',6'-bis(4-carbamoyloxy-1-n-butyl-2,2,6,6-tetramethylpiperidine)
**27)** toluene-2',4'-bis-(4-carbamoyloxy-1-n-propyl-2,2,6,6-tetramethylpiperidine)
**28)** dimethylbis(2,2,6,6-tetramethylpiperidin-4-oxy)silane
**29)** phenyltris(2,2,6,6-tetramethylpiperidin-4-oxy)silane
**30)** tris(1-propyl-2,2,6,6-tetramethylpiperidin-4-yl) phosphite
**30-a)** tris(1-methyl-2,2,6,6-tetramethylpiperidin-4-yl) phosphite
**31)** tris(1-propyl-2,2,6,6-tetramethylpiperidin-4-yl) phosphate
**32)** phenyl bis(1,2,2,6,6-pentamethylpiperidin-4-yl) phosphonate
**33)** 4-hydroxy-1,2,2,6,6-pentamethylpiperidine
**34)** 4-hydroxy-N-hydroxyethyl-2,2,6,6-tetramethylpiperidine
**35)** 4-hydroxy-N-(2-hydroxypropyl)-2,2,6,6-tetramethylpiperidine
**36)** 1-glycidyl-4-hydroxy-2,2,6,6-tetramethylpiperidine
**36-a-1)** 1,2,3,4-tetrakis[2,2,6,6-tetramethylpiperidin-4-yloxycarbonyl]butane
**36-a-2)** bis[2,2,6,6-tetramethylpiperidin-4-yloxycarbonyl]-bis[tridecyloxycarbonyl]butane
**36-b-1)** 1,2,3,4-tetrakis[1,2,2,6,6-pentamethylpiperidin-4-yloxycarbonyl]butane
**36-b-2)** bis[1,2,2,6,6-pentamethylpiperidin-4-yloxycarbonyl]-bis[tridecyloxycarbonyl]butane
**36-c)** 2,2,6,6-tetramethylpiperidin-4-yloxycarbonyl(C₁₅-C₁₇alkane)
**36-d)** 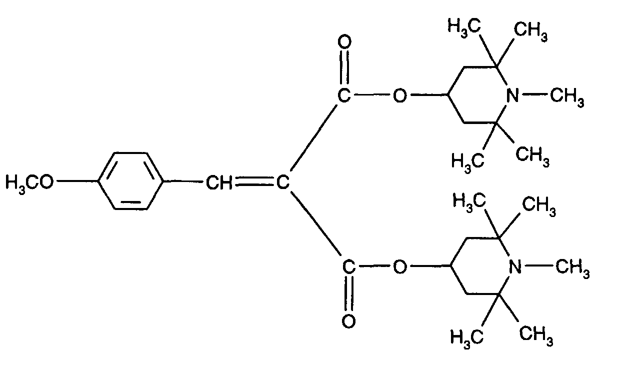
**36-e)** 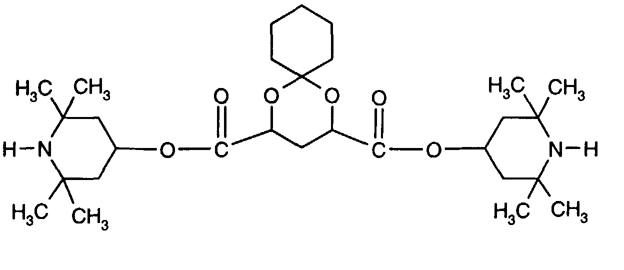
**37)** N,N 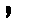 -bis(2,2,6,6-tetramethylpiperidin-4-yl)hexamethylene-1,6-diamine
**38)** N,N 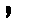 -bis(2,2,6,6-tetramethylpiperidin-4-yl)hexamethylene-1,6-diacetamide
**39)** bis(2,2,6,6-tetramethylpiperidin-4-yl)amine
**40)** 4-benzoylamino-2,2,6,6-tetramethylpiperidine
**41)** N,N 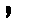 -bis(2,2,6,6-tetramethylpiperidin-4-yl)-N,N 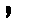 -dibutyladipamide
**42)** N,N 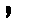 -bis(2,2,6,6-tetramethylpiperidin-4-yl)-N,N 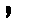 -dicyclohexyl-2-hydroxypropylene-1,3-diamine
**43)** N,N 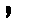 -bis(2,2,6,6-tetramethylpiperidin-4-yl)-p-xylylenediamine
**44)** N,N 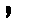 -bis(2,2,6,6-tetramethylpiperidin-4-yl)succinamide
**45)** bis(2,2,6,6-tetramethylpiperidin-4-yl) N-(2,2,6,6-tetramethylpiperidin-4-yl)-β-aminodipropionate
**46)** 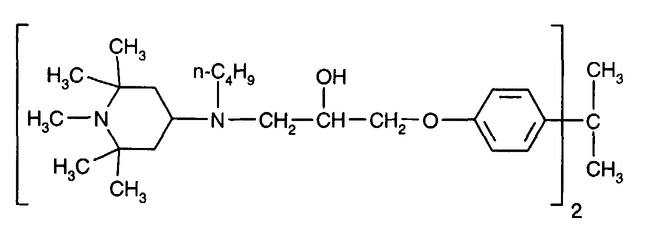
**47)** 4-(bis-2-hydroxyethylamino)-1,2,2,6,6-pentamethylpiperidine
**48)** 4-(3-methyl-4-hydroxy-5-tert-butyl-benzamido)-2,2,6,6-tetramethylpiperidine
**49)** 4-methacrylamido-1,2,2,6,6-pentamethylpiperidine
**49-a-1)** 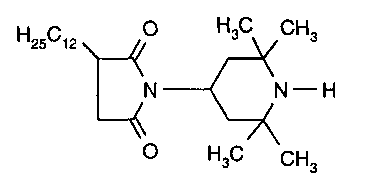
**49-a-2)** 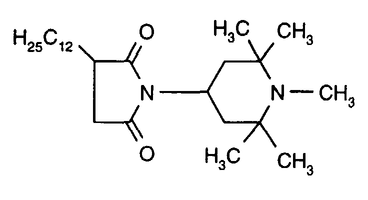
**49-b)** N,N 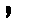 ,N 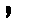 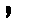 -tris[2,2,6,6-tetramethylpiperidin-4-ylamino(2-hydroxypropylene)]isocyanurate
**49-c)** 2-(2,2,6,6-tetramethylpiperidin-4-ylamino)-2-(2,2,6,6-tetramethylpiperidin-4-ylaminocarbonyl)propane
**49-d)** 1,6-bis[N-(2,2,6,6-tetramethylpiperidin-4-yl)formylamino]hexane
**49-e)** 1-(2,2,6,6-tetramethylpiperidin-4-ylamino)-2-(2,2,6,6-tetramethylpiperidin-4-ylaminocarbonyl)ethane
**50)** 9-aza-8,8,10,10-tetramethyl-1,5-dioxaspiro[5.5]undecane
**51)** 9-aza-8,8,10,10-tetramethyl-3-ethyl-1,5-dioxaspiro[5.5]undecane
**52)** 8-aza-2,7,7,8,9,9-hexamethyl-1,4-dioxaspiro[4.5]decane
**53)** 9-aza-3-hydroxymethyl-3-ethyl-8,8,9,10,10-pentamethyl-1,5-dioxaspiro[5.5]undecane
**54)** 9-aza-3-ethyl-3-acetoxymethyl-9-acetyl-8,8,10,10-tetramethyl-1,5-dioxaspiro[5.5]-undecane
**55)** 2,2,6,6-tetramethylpiperidine-4-spiro-2 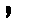 -(1 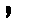 ,3 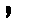 -dioxane)-5 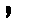 -spiro-5''-(1'',3''-dioxane)-2''-spiro-4'' 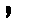 -(2'' 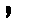 ,2 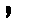 '',6'' 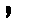 ,6'' 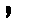 -tetramethylpiperidine)
**56)** 3-benzyl-1,3,8-triaza-7,7,9,9-tetramethylspiro[4.5]decane-2,4-dione
**57)** 3-n-octyl-1,3,8-triaza-7,7,9,9-tetramethylspiro[4.5]decane-2,4-dione
**58)** 3-allyl-1,3,8-triaza-1,7,7,9,9-pentamethylspiro[4.5]decane-2,4-dione
**59)** 3-glycidyl-1,3,8-triaza-7,7,8,9,9-pentamethylspiro[4.5]decane-2,4-dione
**60)** 1,3,7,7,8,9,9-heptamethyl-1,3,8-triazaspiro[4.5]decane-2,4-dione
**61)** 2-isopropyl-7,7,9,9-tetramethyl-1-oxa-3,8-diaza-4-oxospiro[4.5]decane
**62)** 2,2-dibutyl-7,7,9,9-tetramethyl-1-oxa-3,8-diaza-4-oxospiro[4.5]decane
**63)** 2,2,4,4-tetramethyl-7-oxa-3,20-diaza-21-oxodispiro[5.1.11.2]heneicosane
**64)** 2-butyl-7,7,9,9-tetramethyl-1-oxa-4,8-diaza-3-oxospiro[4.5]decane
**65)** 8-acetyl-3-dodecyl-1,3,8-triaza-7,7,9,9-tetramethylspiro[4.5]decane-2,4-dione
**66)** 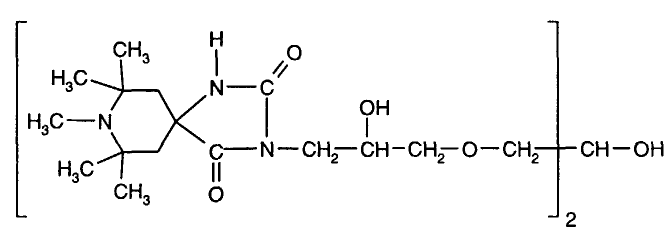
**67)** 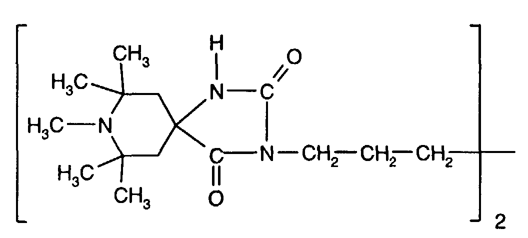
**68)** 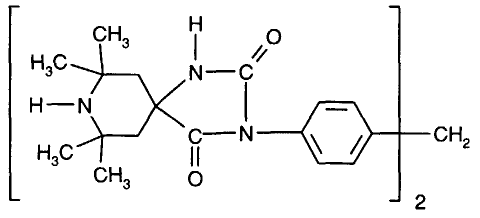
**69-a)** 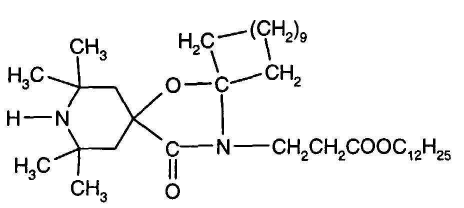
**69-b)** Mixture of 60 % by weight of 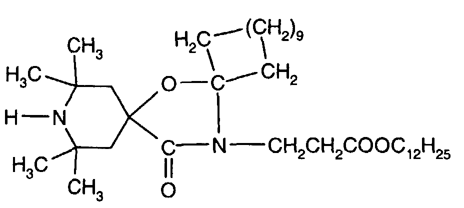 and 40 % by weight of 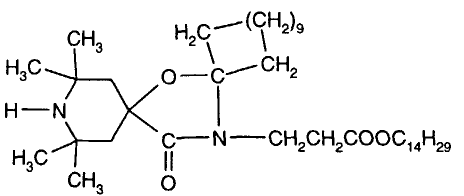
**70)** 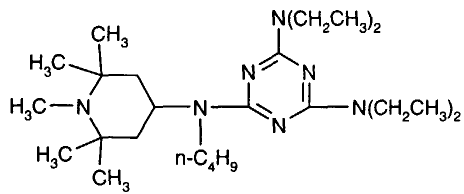
**71)** 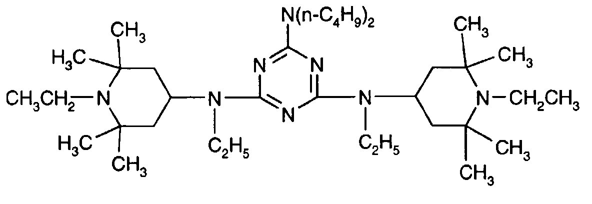
**72)** 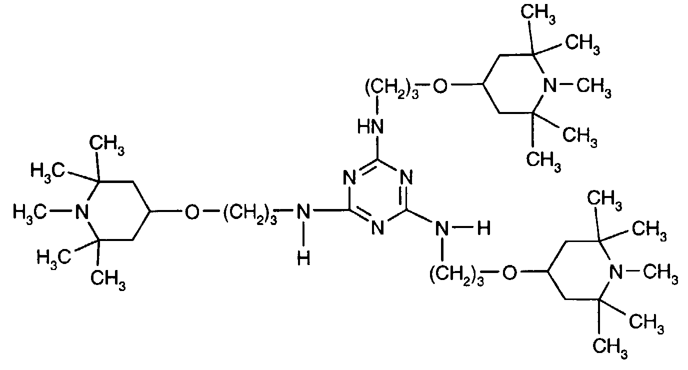
**73)** 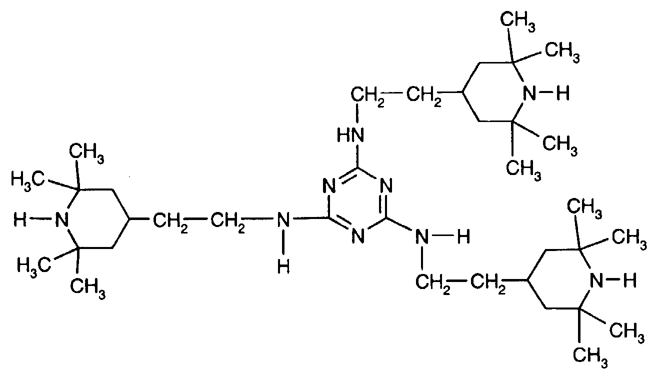
**74)** 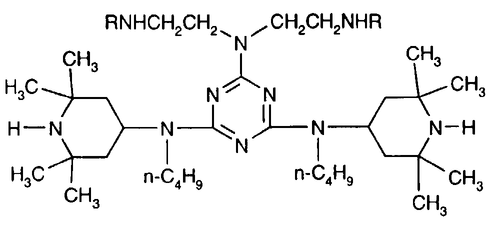 where R is 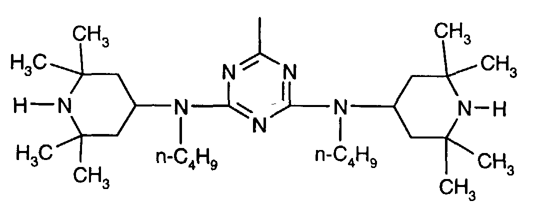
**75)** 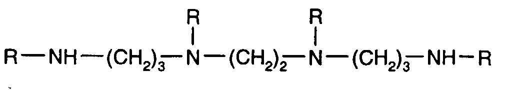 where R has the same meaning as in compound 74.
**76)** 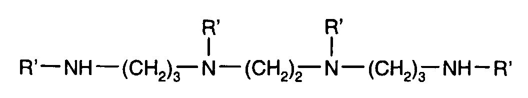 where R 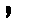 is 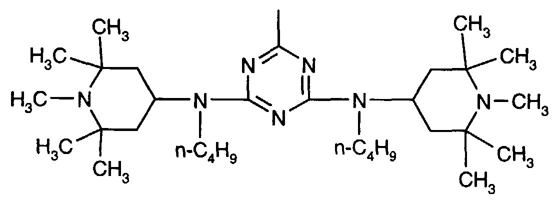
**77)** 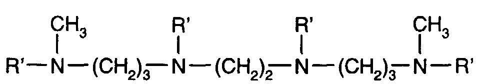 where R 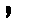 has the same meaning as in compound 76.
**78)** 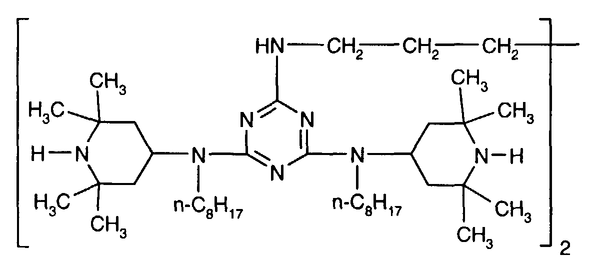
**79)** 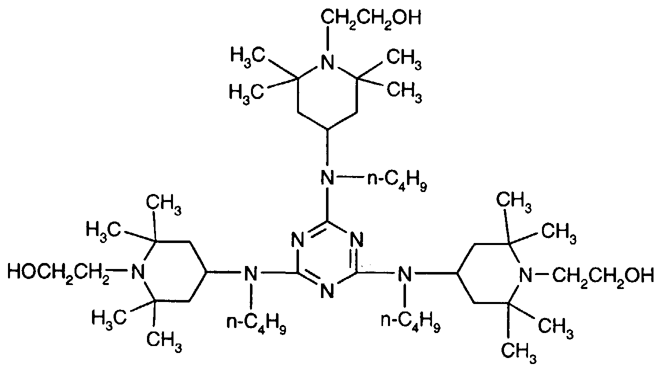
**80)** 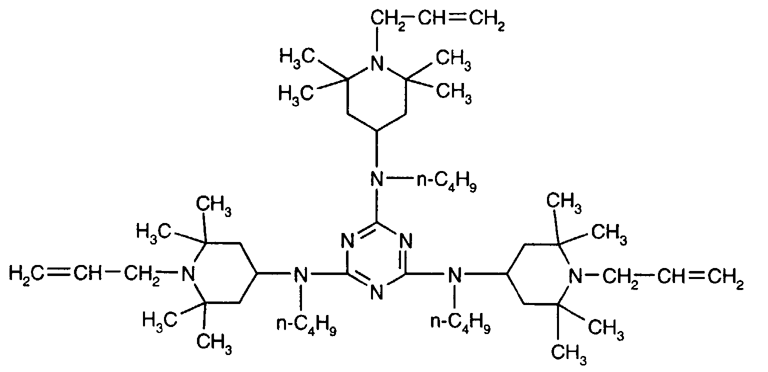
**80-1)** 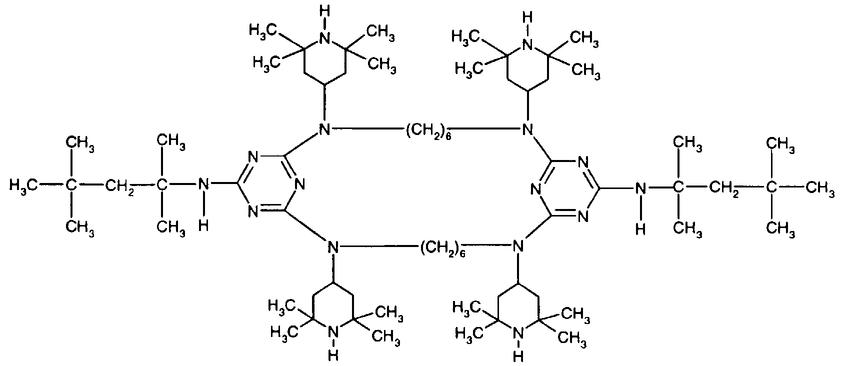
**80-a)** 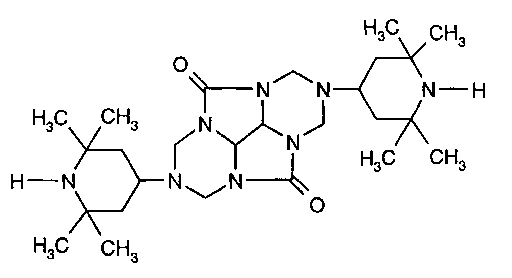
   **In the following compounds 81) to 83), 84-1), 84-2) and 85) to 91, 91-1, 92-1, 92-2, 93 and 94, m**_{**1**} **to m**_{**14**} **is a number from 2 to about 200, preferably 2 to 100, for example 2 to 50, 2 to 40 or 3 to 40 or 4 to 10.**
**81)** 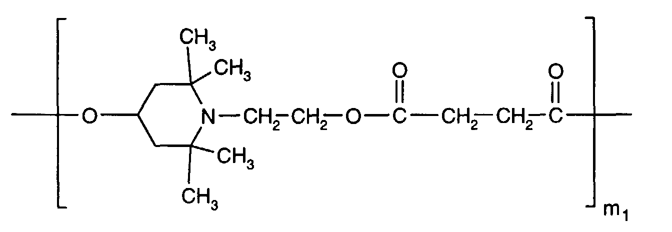
**82)** 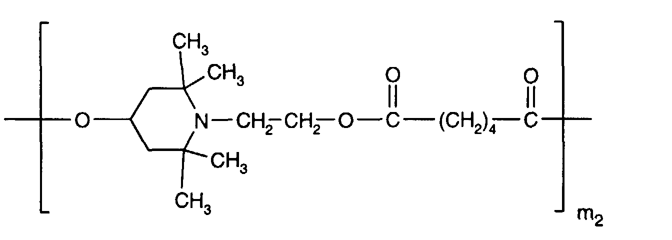
   In the compounds 81 and 82, the end group bonded to the -O- can be, for example, hydrogen or a group -CO-(CH₂)₂-COO-Y or -CO-(CH₂)₄-COO-Y, respectively, with Y being hydrogen or C₁-C₄alkyl and the end group bonded to the diacyl can be, for example, -O-Y or a group 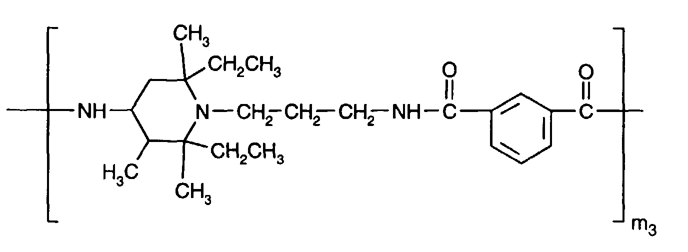
**83)** 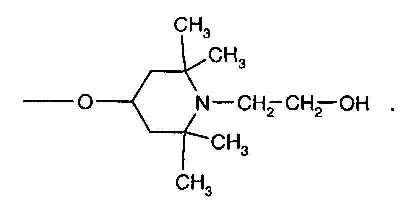
   In the compound 83, the end group bonded to the amino residue can be, for example, a group 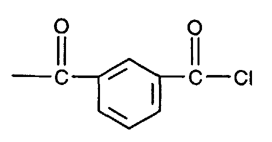 and the end group bonded to the diacyl residue can be, for example, Cl.
**84-1)** 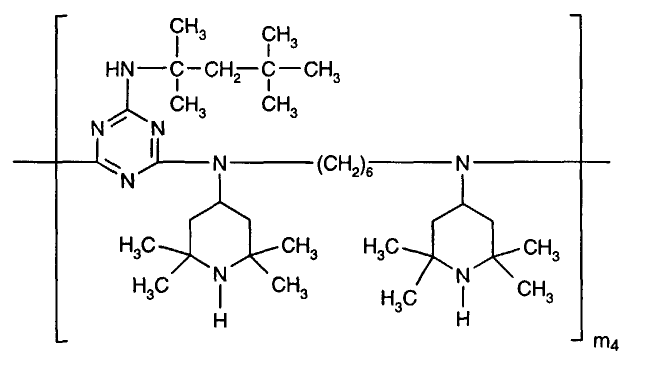
**84-2)** 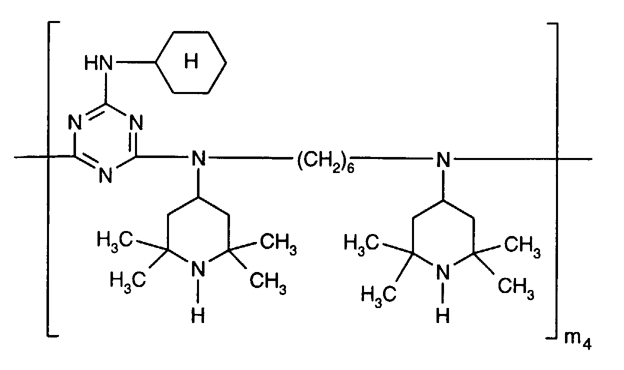
   In the compounds 84-1 and 84-2, the end group bonded to the triazine residue can be, for example, chlorine or a group 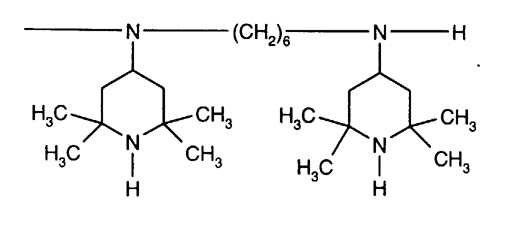 and the end group bonded to the diamino group can be, for example, hydrogen or a group 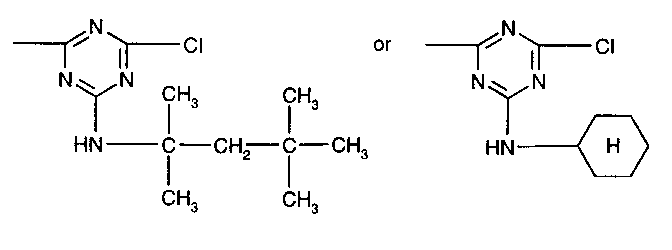
   It may be convenient to replace the chlorine attached to the triazine by e.g. -OH or an amino group. Suitable amino groups are typically: pyrrolidin-1-yl, morpholino, -NH₂, -N(C₁-C₈alkyl)₂ and -NY 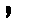 (C₁-C₈alkyl) wherein Y 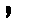 is hydrogen or a group of the formula 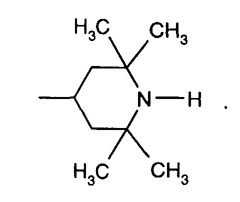
**85)** 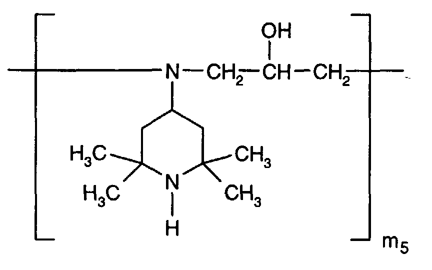
   In the compound 85, the end group bonded to the 2,2,6,6-tetramethylpiperidin-4-ylamino residue can be, for example, hydrogen and the end group bonded to the 2-hydroxypropylene residue can be, for example, 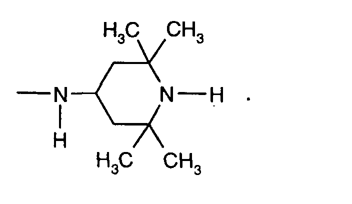
**86)** 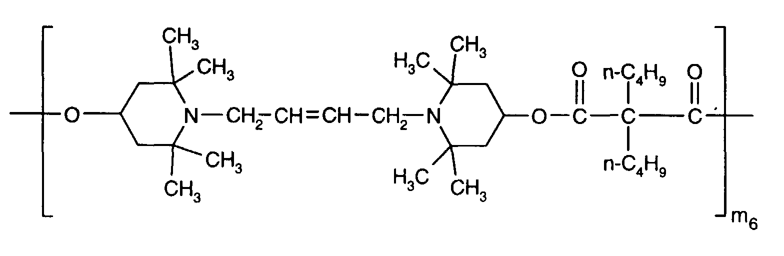
   In the compound 86, the end group bonded to the -O- can be, for example, hydrogen or 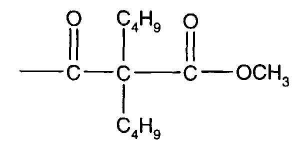 and the end group bonded to the diacyl residue can be, for example, -OCH₃ or Cl.
**87)** 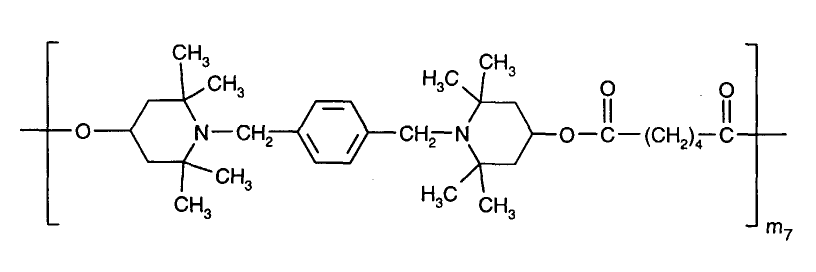
   In the compound 87, the end group bonded to the -O- can be, for example, hydrogen or 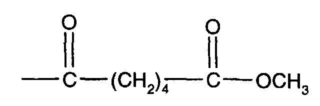 and the end group bonded to the diacyl radical can be, for example, -OCH₃ or Cl.
**88)** 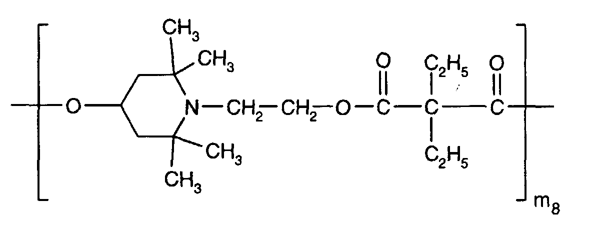
   In the compound 88, the end group bonded to the -O- can be, for example, hydrogen or 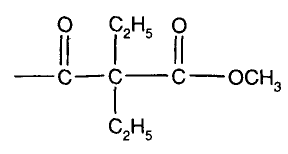 and the end group bonded to the diacyl radical can be, for example, -OCH₃ or Cl.
**89)** 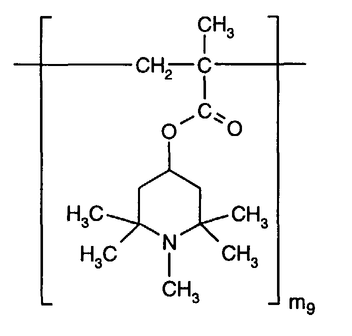
   In the compound 89, the end group bonded to the -CH₂- can be, for example, hydrogen and the end group bonded to the ester residue can be, for example, 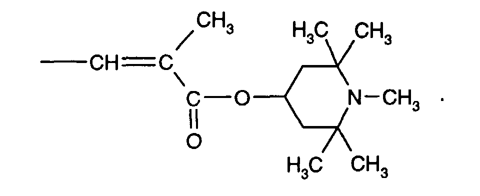
**90)** 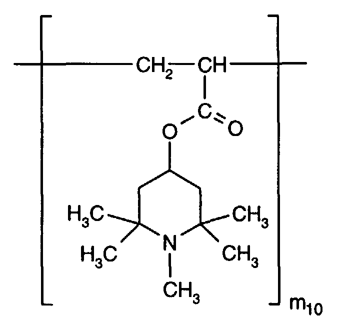
   In the compound 90, the end group bonded to the -CH₂- can be, for example, hydrogen and the end group bonded to the ester residue can be, for example, 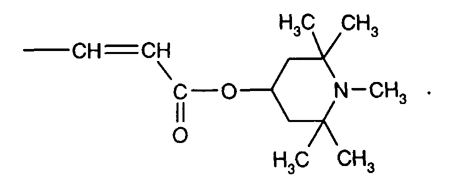
**91)** 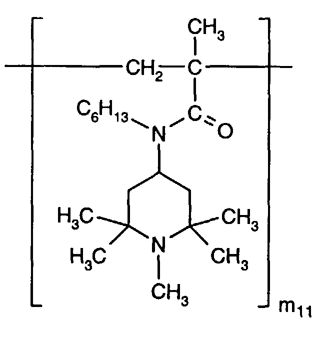
   In the compound 91, the end group bonded to the -CH₂- can be, for example, hydrogen and the end group bonded to the amide residue can be, for example, 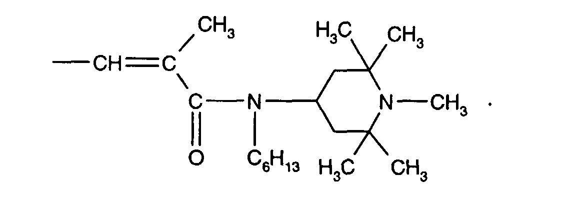
**91-1)** 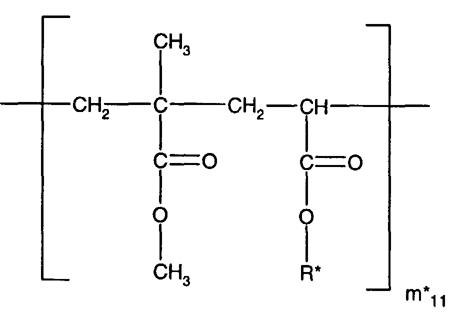 wherein m₁₁* is as defined for m₁₁, the radicals R* independently of one another are ethyl or 2,2,6,6-tetramethylpiperidin-4-yl, with the proviso that at least 50 % of the radicals R* are 2,2,6,6-tetramethylpiperidin-4-yl and the remaining radicals R* are ethyl. In the compound 91-1), the terminal groups are for example hydrogen.
**92-1)** 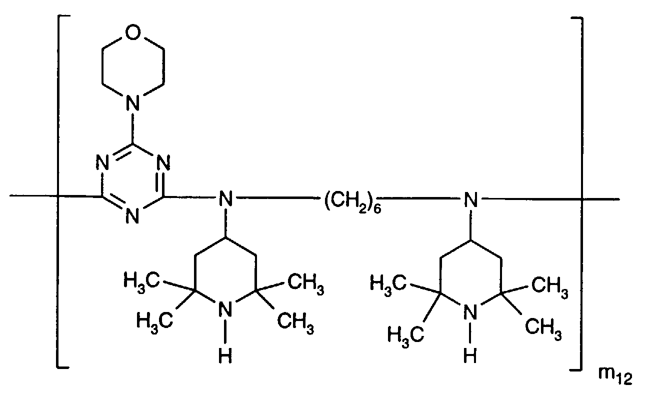
**92-2)** 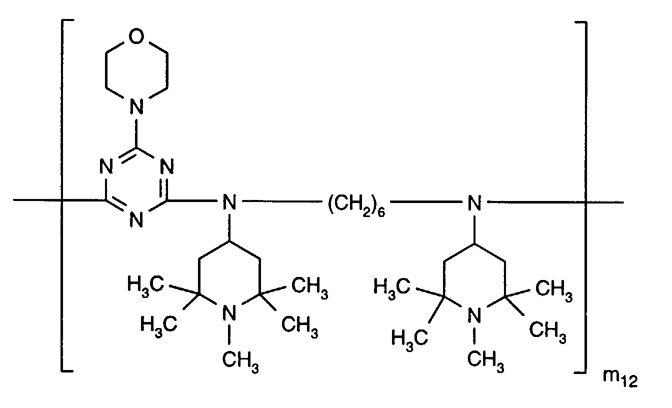
   In the compounds 92-1 and 92-2, the end group bonded to the triazine residue can be, for example, chlorine or a group 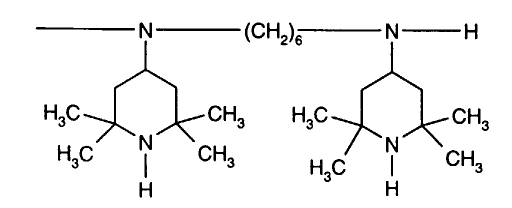 in the compound 92-1, and
   a group 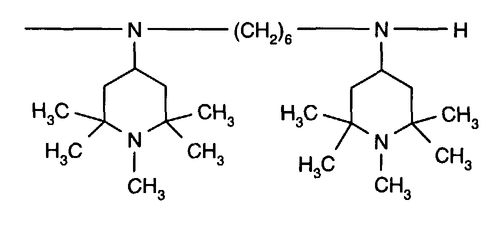 in the compound 92-2,
   and the end group bonded to the diamino residue can be, for example, hydrogen or a group 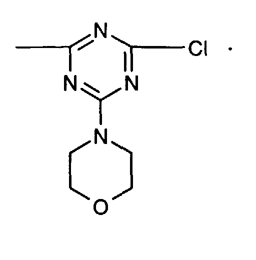
   It may be convenient to replace the chlorine attached to the triazine by e.g. -OH or an amino group. Suitable amino groups are typically: pyrrolidin-1-yl, morpholino, -NH₂, -N(C₁-C₈alkyl)₂ and -NY 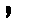 (C₁-C₈alkyl) wherein Y 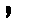 is hydrogen or a group of the formula 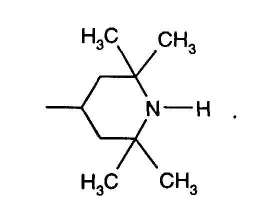
**93)** 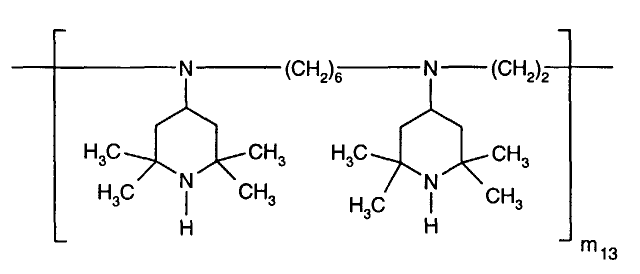
In the compound 93, the end group bonded to the diamino residue can be, for example, hydrogen and the end group bonded to the -CH₂CH₂- residue can be, for example, 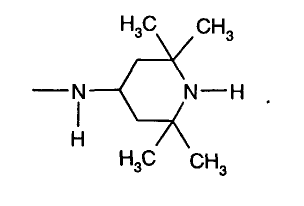
**94)** 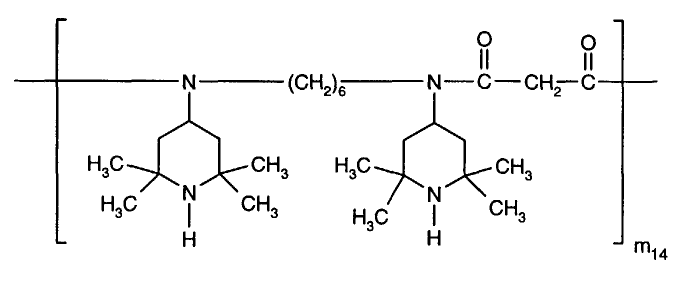
   In the compound 94, the end group bonded to the diamino residue can be, for example, hydrogen and the end group bonded to the diacyl residue can be, for example, Cl.
**95)** 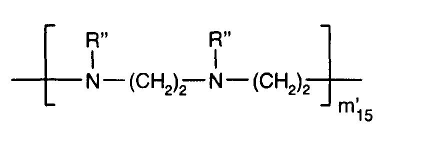 in which R 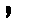 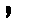 is a group of the formula 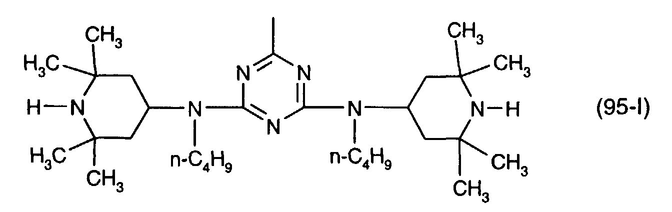 or the chain branching 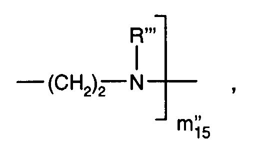
   R 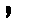 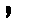 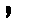 is a group of the formula (95-I), and
   m'₁₅ and m''₁₅ are each a number from 0 to 200, preferably 0 to 100, in particular 0 to 50, with the proviso that m 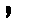 ₁₅ + m''₁₅ is a number from 2 to 200, preferably 2 to 100, in particular 2 to 50. In the compound 95, the end group bonded to the diamino residue can be, for example, hydrogen and the end group bonded to the -CH₂CH₂- group can be, for example, halogen, in particular Cl or Br.
**96)** the compound of the formula (96-I) or (96-II) 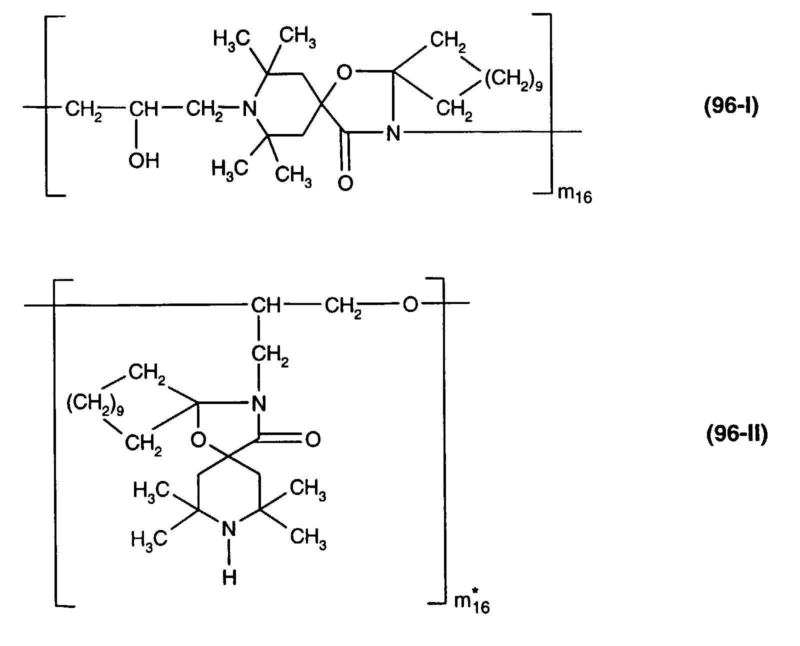 wherein m₁₆ and m₁₆* are a number from 2 to 50.
   During the preparation, the compounds of the formulae (96-I) and (96-II) can be obtained together as a mixture and therefore, can also be employed as such. The (96-I):(96-II) ratio is, for example, from 20:1 to 1:20 or from 1:10 to 10:1.
   In the compounds of the formula (96-I), the terminal group bonded to the nitrogen can be, for example, hydrogen and the terminal group bonded to the 2-hydroxypropylene radical can be, for example, a 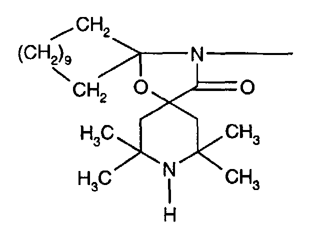 group.
   In the compounds of the formula (96-II), the terminal group bonded to the dimethylene radical can be, for example, -OH, and the terminal group bonded to the oxygen can be, for example, hydrogen. The terminal groups can also be polyether radicals.
**96-a)** 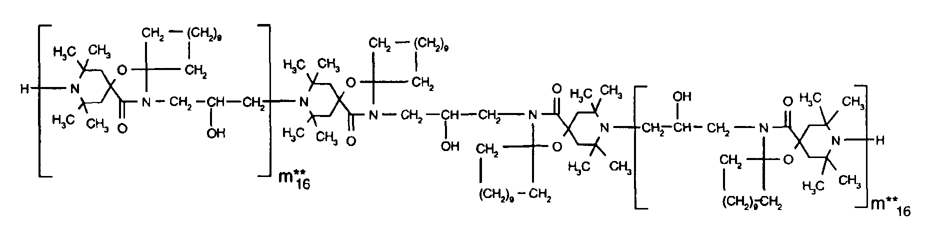 wherein the variables m₁₆** are independently of one another as defined for m₁₆.
**97-1)** 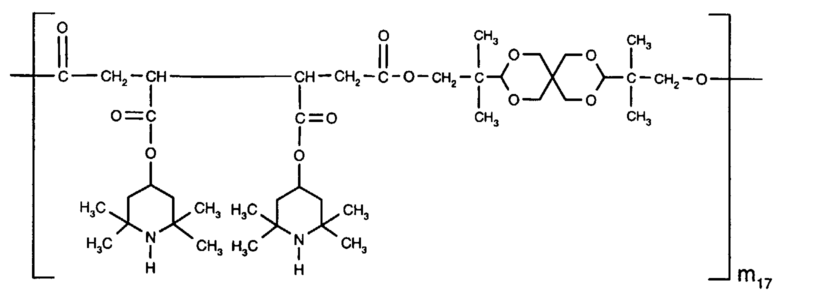
**97-2)** 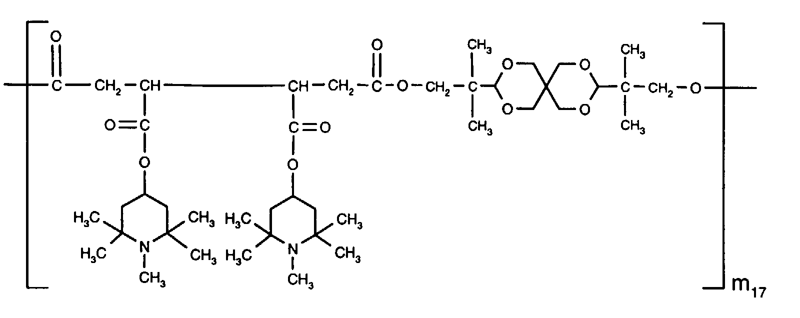
   In the compounds 97-1) and 97-2) the mean value of m₁₇ is 2.5 and the end group bonded to the >C=O group can be, for example, 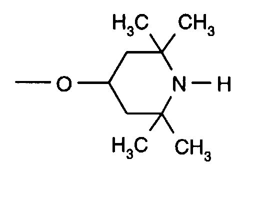 in the compound 97-1) and 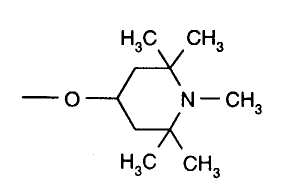 in the compound 97-2); and the end group bonded to the oxygen can be, for example 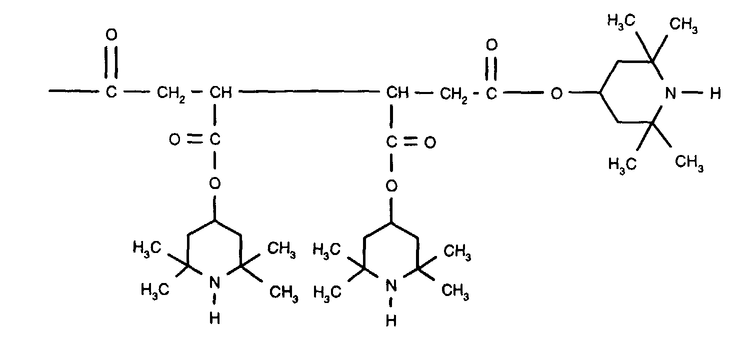 in compound 97-1) and 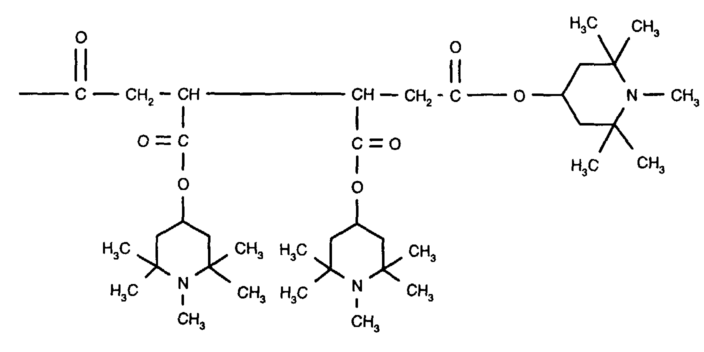 in compound 97-2).
**98)** 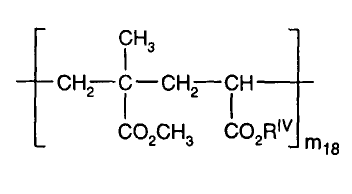 wherein approximately one third of the radicals R^{IV} are -C₂H₅ and the others are a group 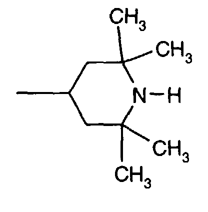 and m₁₈ is a number in the range from 2 to 200, preferably 2 to 100, in particular 2 to 50.
   In the compound 98), the end group bonded to the -CH₂- residue can be, for example, hydrogen and the end group bonded to the -CH(CO₂R^{IV})- residue can be, for example, -CH=CH-COOR^{IV}.
**99-1)** 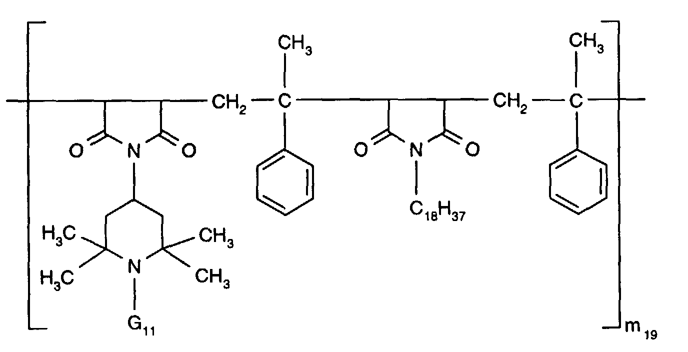
**99-2)** 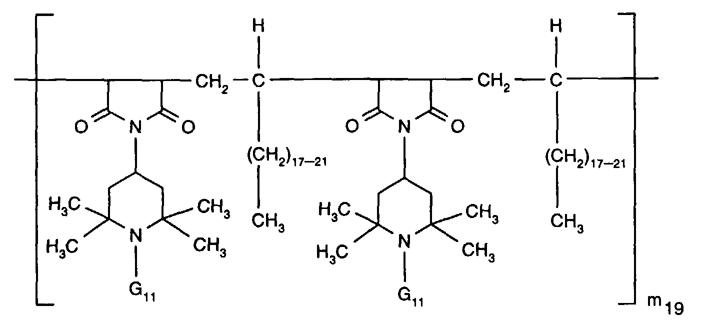
**99-3)** 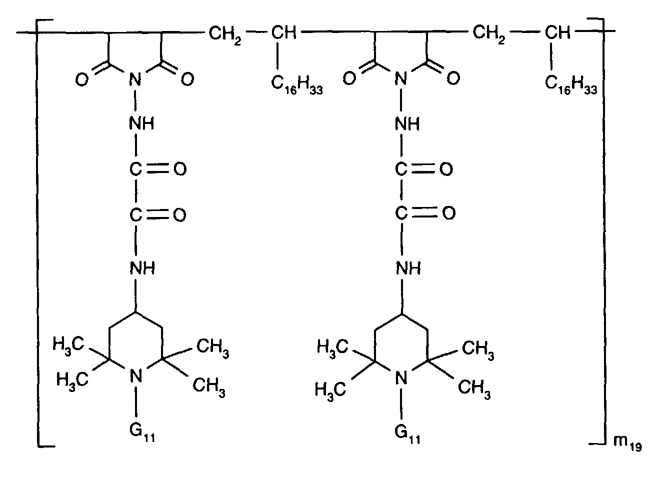
   In the compounds 99-1), 99-2) and 99-3), G₁₁ is hydrogen or methyl, and m₁₉ is a number from 1 to 25.
   In the compounds 99-1), 99-2) or 99-3), the end group bonded to the 2,5-dioxopyrrolidine ring can be, for example, hydrogen, and the other end group can be, for example, a group of the formula 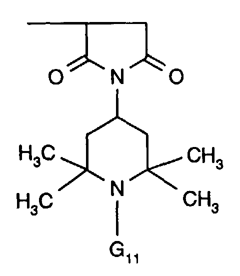 in the compounds 99-1) and 99-2), and a group of the formula 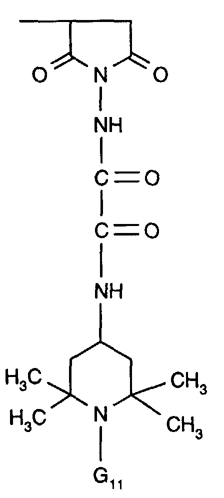 in compound 99-3).
**100)** A product obtainable by reacting a product, obtained by reaction of a polyamine of the formula (100a) with cyanuric chloride, with a compound of the formula (100b) 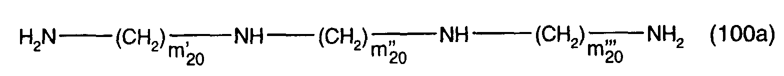 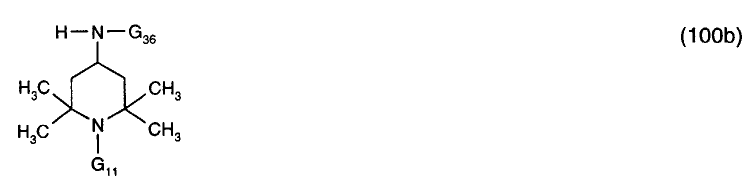
   in which m 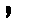 ₂₀, m 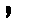 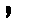 ₂₀ and m 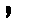 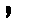 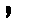 ₂₀, independently of one another, are a number from 2 to 12,
   G₃₆ is hydrogen, C₁-C₁₂alkyl, C₅-C₁₂cycloalkyl, phenyl or C₇-C₉phenylalkyl, and
   G₁₁ is hydrogen or methyl. The product with G₁₁ being hydrogen has the Chemical Abstracts-CAS No. 136 504-96-6.

   In general, the above reaction product can be represented for example by a compound of the formula 100-1, 100-2 or 100-3. It can also be in the form of a mixture of these three compounds. 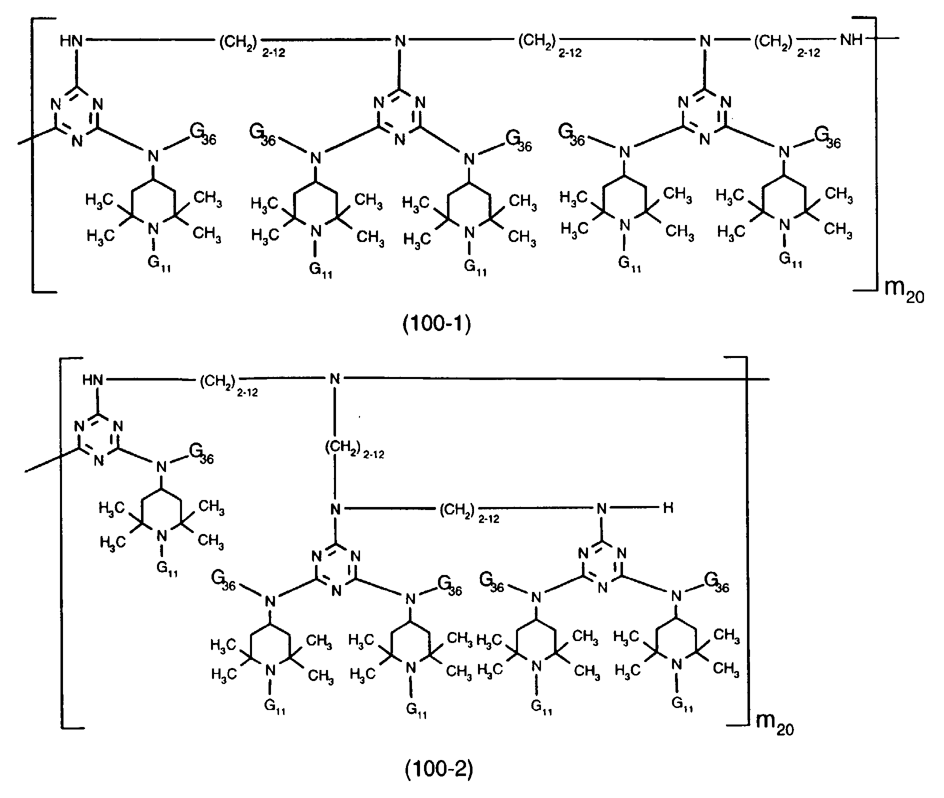 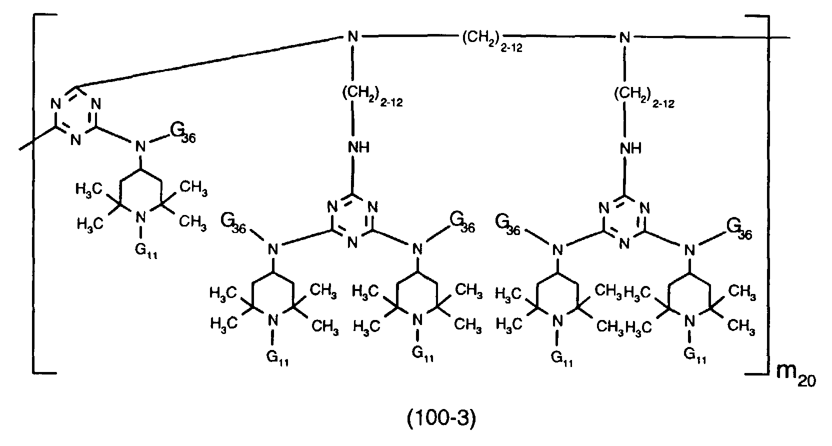
   A preferred meaning of the formula (100-1) is 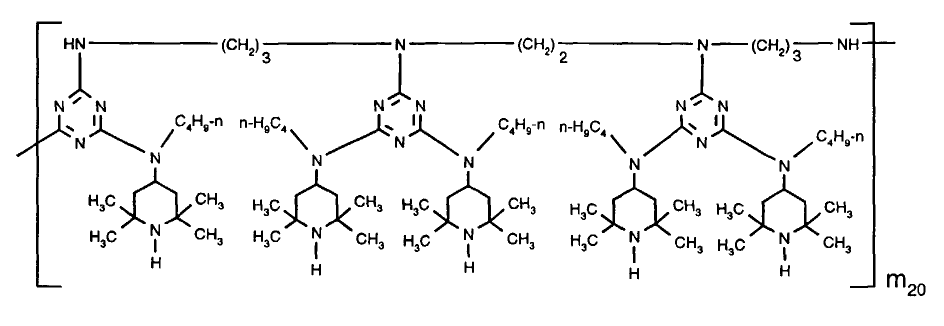
   A preferred meaning of the formula (100-2) is 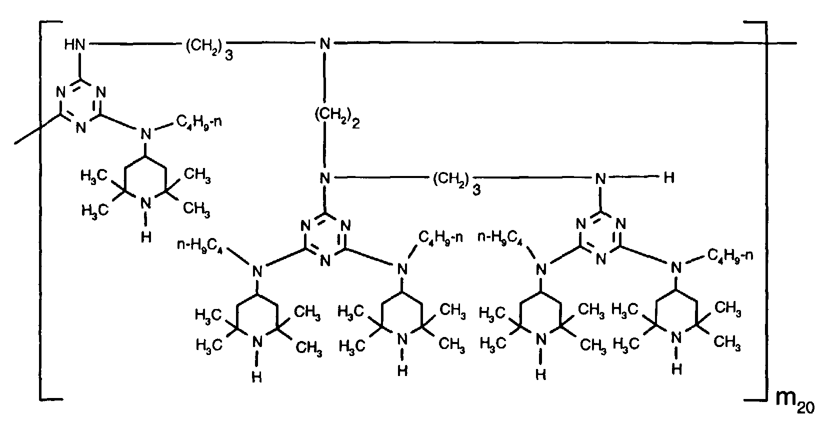
   A preferred meaning of the formula (100-3) is 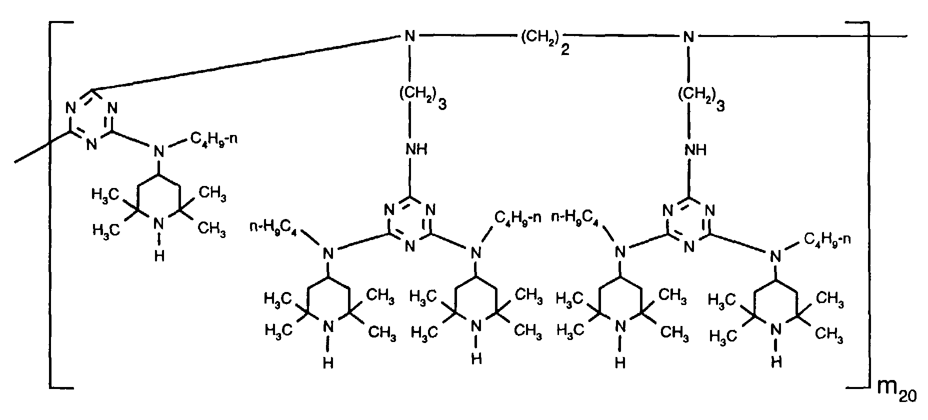
   In the above formulae 100-1 to 100-3, m₂₀ is e.g. 1 to 20, preferably 2 to 20.
**101)** 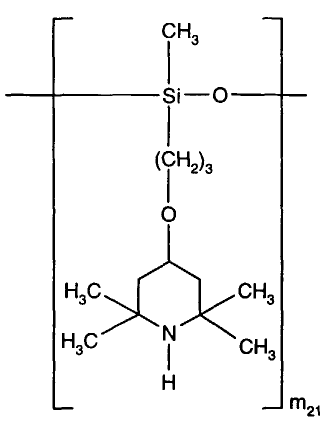 with m₂₁ being a number from 1 to 20.
   In the compound 101), the terminal group bonded to the silicon atom can be, for example, (CH₃)₃Si-O-, and the terminal group bonded to the oxygen can be, for example, -Si(CH₃)₃.
   The compounds 101) can also be in the form of cyclic compounds if m₂₁ is a number from 3 to 10, i.e. the free valences shown in the structural formula then form a direct bond.
**102)** 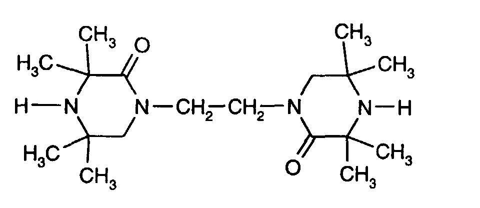
**103)** 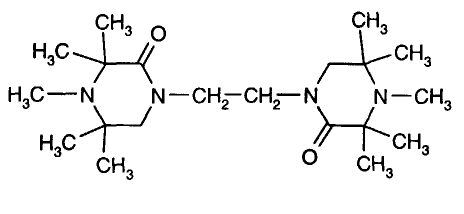
**104)** 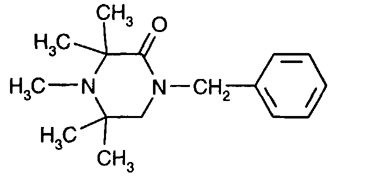
**105)** 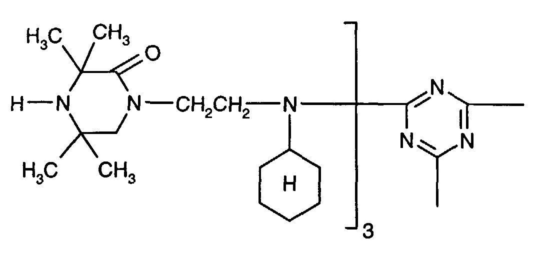
**106)**

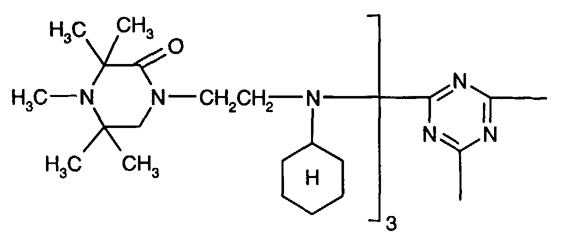

**Component** β**)** can also be one of the compounds described in GB-A-2,301,106 as component I-a), I-b), I-c), I-d), I-e), I-f), I-g), I-h), I-i), I-j), I-k) or I-l), in particular the light stabilizer 1-a-1, 1-a-2, 1-b-1, 1-c-1, 1-c-2, 1-d-1, 1-d-2, 1-d-3, 1-e-1, 1-f-1, 1-g-1, 1-g-2 or 1-k-1 listed on pages 68 to 73 of said GB-A-2,301,106.

The following blends of two commercially available products are particularly useful as stabilizers for organic materials, especially polyolefins:
A) ®CHIMASSORB 2020 and ®TINUVIN 770
B) ®CHIMASSORB 2020 and ®TINUVIN 123
C) ®CHIMASSORB 2020 and ®TINUVIN 765
D) ®CHIMASSORB 2020 and ®TINUVIN 440
E) ®CHIMASSORB 2020 and ®TINUVIN 144
F) ®CHIMASSORB 2020 and ®CHIMASSORB 966
G) ®CHIMASSORB 2020 and ®DASTIB 845
H) ®CHIMASSORB 2020 and ®DIACETAM 5
I) ®CHIMASSORB 2020 and ®GOODRITE UV 3034
J) ®CHIMASSORB 2020 and ®GOODRITE UV 3150
K) ®CHIMASSORB 2020 and ®GOODRITE UV 3159
L) ®CHIMASSORB 2020 and ®CYASORB UV 3581
M) ®CHIMASSORB 2020 and ®CYASORB UV 3604
N) ®CHIMASSORB 2020 and ®HOSTAVIN N 20
O) ®CHIMASSORB 2020 and ®HOSTAVIN N 24
P) ®CHIMASSORB 2020 and ®MARK LA 52
Q) ®CHIMASSORB 2020 and ®MARK LA 57
R) ®CHIMASSORB 2020 and ®MARK LA 62
S) ®CHIMASSORB 2020 and ®MARK LA 67
T) ®CHIMASSORB 2020 and ®SUMISORB TM 61
U) ®CHIMASSORB 2020 and ®UVINUL 4049
V) ®CHIMASSORB 2020 and ®UVINUL 4050 H
W) ®CHIMASSORB 2020 and ®SANDUVOR 3050
X) ®CHIMASSORB 2020 and ®SANDUVOR PR-31
Y) ®CHIMASSORB 2020 and ®UVASIL 299 LM
Z) ®CHIMASSORB 2020 and ®UVASIL 2000 LM

a) ®CHIMASSORB 2020 and ®TINUVIN 622
b) ®CHIMASSORB 2020 and ®CHIMASSORB 944
c) ®CHIMASSORB 2020 and ®CHIMASSORB 119
d) ®CHIMASSORB 2020 and ®CYASORB UV 3346
e) ®CHIMASSORB 2020 and ®CYASORB UV 3529
f) ®CHIMASSORB 2020 and ®DASTIB 1082
g) ®CHIMASSORB 2020 and ®FERRO AM 806
h) ®CHIMASSORB 2020 and ®LICHTSCHUTZSTOFF UV 31
i) ®CHIMASSORB 2020 and ®LUCHEM HA-B18
j) ®CHIMASSORB 2020 and ®HOSTAVIN N 30
k) ®CHIMASSORB 2020 and ®MARK LA 63
l) ®CHIMASSORB 2020 and ®MARK LA 68
m) ®CHIMASSORB 2020 and ®UVINUL 5050 H
n) ®CHIMASSORB 2020 and ®UVASIL 299 HM
o) ®CHIMASSORB 2020 and ®UVASIL 2000 HM
p) ®CHIMASSORB 2020 and ®UVASORB HA 88

Particularly preferred are the mixtures A), L), N), Q), V), W), Y) and Z) as well as the mixtures a), b), d), j), n), o) and p).

The weight ratio of component α) to component β), e.g. the two commercially available compounds listed above, is preferably 20:1 to 1:20, for example 10:1 to 1:10, 5:1 to 1:5, 3:1 to 1:3 or 2:1 to 1:2, in particular 1:1.

The components α) and β) of the stabilizer mixtures described herein can be added to the material to be stabilized, e.g. the polyolefin, individually or mixed with one another. If desired, the individual components of the stabilizer mixture can be mixed with one another in the melt (melt blending) before incorporation into the material to be stabilized.

The mixture containing components α) and β) is preferably present in the material to be stabilized in an amount of 0.01 to 10% by weight, relative to the material to be stabilized. An amount of 0.01 to 5% by weight or 0.05 to 2% by weight, in particular 0.05 to 0.5% by weight is especially preferred.

The mixtures described herein can be added, for example, to polymeric materials before, during or after the polymerization or crosslinking of the said materials. Furthermore, they can be incorporated in the polymeric materials in the pure form or encapsulated in waxes, oils or polymers.

In general, the stabilizer mixtures can be incorporated in the polymeric materials by various processes, such as dry mixing in the form of powder, or wet mixing in the form of solutions or suspensions or also in the form of a masterbatch which contains the stabilizer mixture in a concentration of 2.5 to 25 % by weight; in such operations, the polymer can be used in the form of powder, granules, solutions, suspensions or in the form of latices.

The materials containing the stabilizer mixtures described herein can be used for the production of mouldings, films, tapes, monofilaments, fibres, surface coatings and the like.

**Component** α**)** of the above described stabilizer mixtures can also be one of the following products:
1) A compound of the formula (I) described in GB-A-2,325,667, in particular the compound of Example 1D or Example 2 to 8.
2) A product as described in claim 1, 19, 20, 25 or 29 of WO-A-98/54176 or the product disclosed in Example 1A, 1B or 2 of said WO-A-98/54176.
3) A product as described in claim 1, 18, 19, 25 or 29 of WO-A-98/54177 or the product disclosed in Example I-1, I-2, 1, 1-A, 1-B, 2-A or 2-B of said WO-A-98/54177.
4) A product as described in claim 1, 17 or 18 of WO-A-98/54175 or the product disclosed in Example 1, 2, 2-A, 2-B, 3-A, 3-B, 4, 4-A, 4-B of said WO-A-98/54175.
5) A compound of the formula (I) described in WO-A-98/54173, in particular the product of Example 1, 2, 3 or 4.
6) A compound of the formula (I) described in WO-A-98/54174, in particular the compound of Example V-1 or V-2.
7) A compound of the formula (I) described in EP-A-850,938, in particular the compound of Example A, 1, 2, 3, 4, 5, 6 or 7.

## Claims

1. A mixture comprising two different sterically hindered amines one of which is a compound of the formula 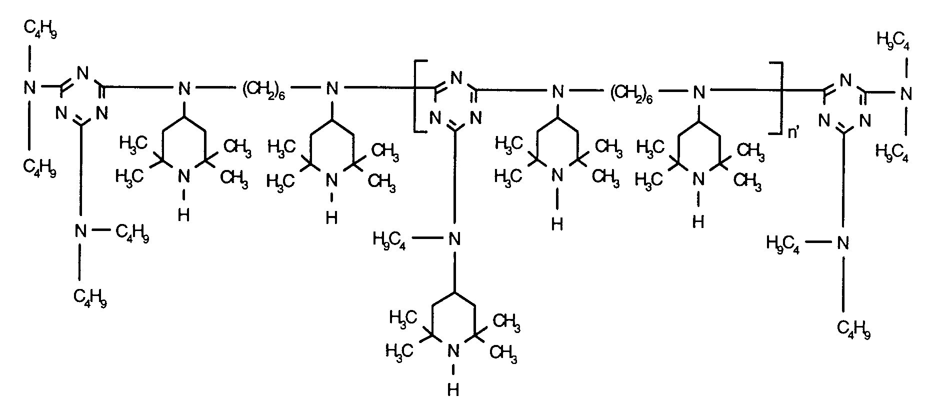 wherein n 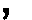 is a number from 2 to 14.
